# EUROPÄISCHE PATENTANMELDUNG

(11) **EP 4 710 881 A1**
(43) Veröffentlichungstag der Anmeldung: **18.03.2026**
(21) Anmeldenummer: 25198729.3
(22) Anmeldetag: 28.08.2025
(51) Int. Cl.: A61B 18/14, A61B 18/00

(54) **LAPAROSKOPISCHER APPLIKATOR UND SYSTEM ZUR LAPAROSKOPISCHEN ABLATION**

(30) Priorität: 11.09.2024 DE 102024126105; 12.11.2024 US 202418944629
(71) Anmelder: Stockert GmbH, 79111 Freiburg im Breisgau (DE)
(72) Erfinder: KEES, Fabian, 79110 Freiburg im Breisgau (DE); HO, Patty Ky, 79249 Merzhausen (DE); STORK, David, 79114 Freiburg (DE); KOCH, Fritz, 79100 Freiburg (DE)
(74) Vertreter: Frenkel, Matthias Alexander

(57) **Zusammenfassung**

Die vorliegende Erfindung beschreibt eine Vorrichtung zur Anwendung in der laparoskopischen Chirurgie und ein System zur laparoskopischen Ablation.

## Beschreibung

Die vorliegende Erfindung ordnet sich im Bereich der minimal-invasiven medizinischen Verfahren ein, mit einem speziellen Fokus auf der Ablationstechnik. Sie konzentriert sich insbesondere auf die irreversible Elektroporation (IRE) von Gewebe, eine fortschrittliche Methode, die in der interventionellen Medizin zur gezielten Behandlung von Gewebe eingesetzt wird. Diese Technik stellt einen bedeutenden Fortschritt in der medizinischen Therapie dar und wird bereits gezielt für die Ablation von Herzgewebe eingesetzt, unter besonderer Berücksichtigung der Gewebeselektivität, reduzierter Behandlungszeiten und der Minimierung von Risiken konventioneller Behandlungsmethoden.

In den vergangenen Jahren hat sich die Behandlung von Gewebe mittels gepulster elektrischer Felder als eine zunehmend relevante klinische Technik etabliert. Die Verwendung von kurzen elektrischen Hochspannungs-Pulsen und die damit verbundenen hohen elektrischen Feldstärken, die auf das Gewebe einwirken, sind jedoch bereits seit mehr als vier Jahrzehnten Gegenstand intensiver Forschung. Diese Anwendungsmethode wird als nicht-thermisches Verfahren kategorisiert, da sie auf der Abgabe kurzer Pulse mit hoher Spannungsamplitude basiert, die ein lokal starkes elektrisches Feld im Bereich von bis zu mehreren Tausend Volt pro Zentimeter zwischen aktiven Elektrodenpaaren erzeugen. Diese Feldstärke führt zur kurzzeitigen Bildung von Poren in den Zellmembranen. Überschreitet das elektrische Feld einen bestimmten Schwellenwert, der für die Bildung von Poren in den Lipiddoppelschichten der Zellmembranen erforderlich ist, und ist das Gewebe diesem Feld über einen kritischen Zeitraum ausgesetzt, sterben die Zellen durch Apoptose.

Zur Behandlung von Herzrhythmusstörungen, wie z. B. Vorhofflimmern hat sich die gepulste Feldablation bereits als vielversprechende Behandlungsmethode erwiesen. Die Neuromodulation von Nerven mittels gepulster elektrischer Hochspannungssignale ist dagegen noch Gegenstand zahlreicher Forschungsprojekte. Ein erhebliches Forschungsfeld deckt dabei die renale Denervation ab. Dabei handelt es sich um eine Methode zur Unterdrückung erhöhter sympathischer Aktivität zur Behandlung von Bluthochdruck oder anderen Herz-Kreislauf-Erkrankungen. Der derzeitige Therapieansatz ist ein minimalinvasiver Eingriff, bei dem mithilfe eines speziellen Katheters die Nervenleitungen rund um die Nierenarterien unterbunden werden. Der Katheter wird dabei in die Nierenarterie eingeführt, und die außerhalb der Arterie verlaufenden Nervenfasern werden durch die Gefäßwand hindurch mittels thermischer oder chemischer Ablation verödet.

Auch wenn der zuvor beschriebene minimal-invasive endovaskuläre Katheter-basierte Ansatz zur Neuromodulation als vielversprechende Behandlungsmethode angesehen wird, so ließen einige der durchgeführten Studien Zweifel an der Wirksamkeit aufkommen. Da beispielsweise die renalen sympathischen Nervenfasern außerhalb der Arterie liegen und somit Katheter-basiert nicht direkt erreicht werden können, kann ein Laparoskopie-basierter Eingriff zur perivaskulären Neuromodulation eine vielversprechende alternative Prozedur darstellen. Auf diese Weise können die Nervenfasern direkt in Kontakt mit einem laparoskopischen Ablations-Instrument gebracht werden und damit die Wahrscheinlichkeit für eine erfolgreiche Behandlung erhöht werden.

Die irreversible Elektroporation (IRE) ist im Wesentlichen ein nicht-thermisches Verfahren, welches nur eine geringe elektrische Energie nutzt und damit eine Erhöhung der Gewebetemperatur um nur einige °C bewirkt. Dies grenzt es deutlich von der konventionellen RF-Ablation (RF: Radiofrequenz) ab, bei der die Gewebetemperatur um 20 bis 70 °C steigt und Zellen durch Hitze zerstört werden. Bei der IRE werden in der Regel bipolare Pulse eingesetzt, d.h. eine Kombination aus positiven und negativen elektrischen Pulsen, um Muskelkontraktionen, die üblicherweise bei Anwendung von Gleichspannung entstehen, weitestgehend zu vermeiden. Diese Pulse können zwischen zwei bipolaren Elektroden eines Applikators oder zwischen einer Applikatorelektrode und einer Körperoberflächenelektrode, die üblicherweise auf dem Rücken des Patienten angebracht wird, angelegt werden.

Damit die IRE-Pulse Poren im Gewebe erzeugen, muss die durch die Pulse definierte elektrische Feldstärke E am Gewebe zwischen einem Paar aus mindestens zwei Elektroden einen gewebeabhängigen Schwellenwert Eth überschreiten. Beispielsweise liegt der Schwellenwert für Herzzellen bei etwa 500 V/cm, während er für Knochen bei 3000 V/cm liegt. Diese Unterschiede in den Schwellenwerten ermöglichen eine selektive Anwendung von IRE in verschiedenen Geweben. Um die erforderliche Feldstärke zu erreichen, hängt die an ein Elektrodenpaar anzulegende Spannung sowohl vom Zielgewebe als auch vom Abstand zwischen den Elektroden und von der Elektrodengröße selbst ab. Gleichermaßen beeinflussen diese Parameter auch den thermischen Energieeintrag während der Ablation und damit die Temperaturspitzen, die an dem zu behandelnden Gewebe auftreten können. Die angelegten Spannungen können mehrere Kilovolt erreichen und liegen damit deutlich über den für die thermische RF-Ablation typischen Spannungen von 10-200 V.

Am Markt etablierte Systeme zur Neuromodulation von Nerven und speziell zur renalen Denervation nutzen einen transvaskularen Katheter-basierten Ansatz, entweder mittels RF-Energie oder Ultraschall. Ein genereller Nachteil der transvaskularen Neuromodulation besteht jedoch darin, dass zur Verödung der äußeren Nervenstränge durch die Gefäßwand hindurch abladiert werden muss. Um dem entgegenzuwirken, sieht ein alternatives Behandlungsverfahren den Einsatz von laparoskopischen Applikatoren auf. Diese werden in den Torso des Patienten eingeführt und können auf diese Weise die Nerven um die Arterie herum perivaskulär von außen adressieren. Damit kann der notwendige Energieeintrag in das Gewebe reduziert werden, wodurch man sich einerseits eine erhöhte Wahrscheinlichkeit für eine erfolgreiche Neuromodulation verspricht und gleichzeitig das Risiko für eine Beschädigung des Gefäßes minimieren kann.

Laparoskopische Systeme für eine solche perivaskuläre Neuromodulation, beispielsweise zur renalen Denervation, sind aus der EP 4335397 A1 bekannt. Darin wird eine Ablations-Vorrichtung einschließlich einer Elektrodeneinheit beschrieben, welches ein schlauchförmiges Gewebe-Stück umschließen kann, um über ein thermisches Ablationsverfahren (bspw. Radiofrequenz-Ablation) Nerven zu denervieren.

Es besteht ein Bedarf nach verbesserten Ablationsansätzen.

Die vorliegende Erfindung betrifft die laparoskopische Ablation, insbesondere die Laparoskopie-basierte Neuromodulation.

Hierfür werden eine Vorrichtung nach Anspruch 1 und ein System nach Anspruch 15 vorgeschlagen.

Gemäß einem ersten Aspekt wird eine Vorrichtung zur Anwendung in der laparoskopischen Chirurgie vorgeschlagen. Die Vorrichtung weist einen Schaft auf. Der Schaft weist ein distales und ein proximales Ende auf. Die Vorrichtung weist eine Halteeinheit auf, die an dem distalen Ende des Schafts angeordnet ist. Die Halteeinheit ist dazu eingerichtet, ein Gewebe zu halten und/oder loszulassen. Die Vorrichtung weist zumindest zwei in/an/mit der Halteeinheit integrierte Elektroden auf. Die Elektroden können unmittelbar und/oder fest mit der Halteeinheit verbunden sein. Die Vorrichtung weist eine an/in dem Schaft angeordnete und mit der Halteeinheit, beispielsweise mittelbar, verbundene Kraftleitungseinheit auf, die dazu eingerichtet ist, relativ zu dem Schaft bewegt zu werden. Die Vorrichtung weist eine elektrische Leitung auf, die in dem Schaft angeordnet und mit den zumindest zwei Elektroden, insbesondere elektrisch leitend, verbunden und dazu eingerichtet ist, ein über das proximale Ende des Schafts zu empfangendes elektrisches Signal an die zumindest zwei Elektroden weiterzuleiten, insbesondere um auf/in/an einem gehaltenen Gewebe eine irreversible Elektroporation durchzuführen.

Die Vorrichtung kann auch als ein laparoskopischer Applikator bezeichnet werden. Die Vorrichtung kann einen Trokar aufweisen, der dazu eingerichtet ist, den Schaft durch/in den Torso eines Patienten einzuführen.

Die Vorrichtung kann eine Gelenkanordnung aufweisen. Die Gelenkanordnung kann zwischen der Halteeinheit und dem distalen Ende des Schafts angeordnet sein. Die Gelenkanordnung kann an dem distalen Ende des Schafts angeordnet und/oder, insbesondere unmittelbar, damit verbunden sein. Die Gelenkanordnung kann dazu eingerichtet sein, das distale Ende des Schaftes mit der Halteeinheit, insbesondere beweglich, zu verbinden. Eine Bewegung der Kraftleitungseinheit kann zu einer Bewegung der Halteeinheit und/oder zu dem Halten und/oder Loslassen des Gewebes mittels der Kraftleitungseinheit führen. Eine Bewegung der Kraftleitungseinheit kann zu einer Bewegung der Gelenkanordnung führen, die wiederum zu einer Bewegung der Halteeinheit und/oder zu dem Halten und/oder Loslassen des Gewebes führt/führen kann.

Die Halteeinheit kann als ein Fingergreifer, insbesondere als ein Zwei- oder Drei-Fingergreifer, ausgebildet sein. Die Kraftleitungseinheit kann als Zug- und Druckstab ausgebildet sein. Eine Bewegung des Zug- und Druckstabs, insbesondere relativ zu dem Schaft, kann zu einem Öffnen oder Schließen des Fingergreifers führen, um das Gewebe zu greifen/klemmen oder loszulassen.

Die Vorrichtung kann eine Längs-, Quer und Hochachse aufweisen. Der Schaft kann sich entlang der Längsachse erstrecken. Der Zug- und Druckstab kann dazu eingerichtet sein, sich entlang der Längsachse zu bewegen. Eine Bewegung des Zug- und Druckstabs entlang der/einer, insbesondere Längs-, Achse führt zu einer Öffnungsbewegung des Fingergreifers entlang einer Hoch- und/oder Querachse, insbesondere in der Hoch- und Längsachsenebene.

Die Finger des Fingergreifers können jeweils eine Innfläche aufweisen, die, insbesondere in einem Schließ-Zustand des Fingergreifers, zu dem Gewebe orientiert sein kann und/oder mit einem gehaltenen Gewebe in Kontakt stehen kann. In/auf mindestens eine von zumindest zwei der Innenflächen kann jeweils eine der zumindest zwei, insbesondere Ablations-, Elektroden, insbesondere eine von einer Vielzahl von Elektroden, integriert sein. Zumindest zwei Elektroden der Vielzahl von Elektroden können als Ablationselektroden ausgebildet sein. Zumindest drei der Vielzahl von Elektroden können als Messelektroden ausgebildet sein.

Die Halteeinheit kann als eine Ansaugeinheit ausgebildet sein. Eine Bewegung der Kraftleitungseinheit kann zu einem Abwinkeln, beispielsweise einem beliebigen Einstellen eines Winkels, insbesondere zwischen inklusive 0° und 90°, insbesondere in einer Hoch- und Längsachsebene, der Ansaugeinheit gegenüber dem Schaft führen. Die Ansaugeinheit kann dazu eingerichtet sein, ein Gewebe anzusaugen und/oder sich an dem Gewebe zu fixieren oder sich vom Gewebe zu lösen.

Die Ansaugeinheit kann eine Ansaugwanne aufweisen. Die Ansaugeinheit kann zumindest ein Ansaugloch aufweisen, das in der Ansaugwanne angeordnet ist. Die Ansaugeinheit kann einen Ansaugkanal aufweisen, der mit dem zumindest einen Loch verbunden ist. Die Ansaugeinheit kann einen an einem Ende des Ansaugkanals vorgesehenen Ansauganschluss aufweisen. Über den Ansauganschluss kann eine externe Vakuumpumpe angeschlossen werden, um einen Unterdruck in der Ansaugwanne zu erzeugen und das Gewebe an/in die Ansaugwanne anzusaugen oder durch Ausschalten der externen Vakuumpumpe oder Reduzierung des Unterdrucks in der Ansaugwanne sich vom Gewebe zu lösen.

Die Ansaugwanne kann entlang einer Hochachse oval, insbesondere stadionförmig oder kreisrund, ausgebildet sein. Die zumindest zwei, insbesondere Ablations-, Elektroden können entlang der Hochachse und/oder quer zu der Hochachse in der Ansaugwanne angeordnet sein.

Die Ansaugwanne kann eine Wannen-Breite und Wannen-Länge aufweisen. Die Ansaugwanne kann ein flexibles Material aufweisen. Beispielsweise kann sich dadurch die Breite der Ansaugwanne (etwas) anpassen, sobald Gewebe angesaugt wird. Damit kann die Ansaugwanne sich an eine Struktur und/oder Form des anzusaugenden Gewebes anpassen.

Die Ansaugwanne kann entlang einer Hochachse oval, insbesondere stadionförmig oder kreisrund, ausgebildet sein. Zumindest drei, insbesondere Mess-, Elektroden können, beispielsweise zusätzlich zu den zumindest zwei Elektroden, entlang der Hochachse und/oder quer zu der Hochachse in der Ansaugwanne, insbesondere in einem ersten Elektrodenabstand zu den zumindest zwei Elektroden beabstandet, angeordnet sein. Die zumindest zwei/drei Elektroden können entlang der Hochachse in einem oberen/unteren Bereich (in) der Ansaugwanne angeordnet sein, während die zumindest drei/zwei Elektroden in einem unteren/oberen Bereich (in) der Ansaugwanne quer zu der Hochachse angeordnet sein können. Ausführlicher formuliert können die zumindest zwei Elektroden (z. B. Ablations-Elektroden) entlang der Hochachse in einem oberen Bereich (in) der Ansaugwanne angeordnet sein, während die zumindest drei Elektroden (z. B. Mess-Elektroden) in einem unteren Bereich (in) der Ansaugwanne quer zu der Hochachse angeordnet sein können. Alternativ können die zumindest drei Elektroden (z. B. Mess-Elektroden) entlang der Hochachse in einem unteren Bereich (in) der Ansaugwanne angeordnet sein, während die zumindest zwei Elektroden (z. B. Ablations-Elektroden) in einem oberen Bereich (in) der Ansaugwanne quer zu der Hochachse angeordnet sein können.

Die Halteeinheit kann als ein Schalengreifer, insbesondere ein Zwei-Halbschalengreifer, ausgebildet sein. Eine Bewegung der Kraftleitungseinheit kann zu einem Abwinkeln, insbesondere einem beliebigen Einstellen eines Winkels, insbesondere zwischen inklusive 0° und 90°, insbesondere in einer Quer- und Längsachsebene, des Schalengreifers gegenüber dem Schaft und/oder zum Öffnen und/oder zum Schließen des Schalengreifers führen.

Der Schalengreifer kann sich entlang einer Querachse erstrecken/ausdehnen und/oder eine maximale Ausdehnung aufweisen. Der Schalengreifer kann dazu eingerichtet sein, sich entlang einer Hochachse zu öffnen oder zu schließen. In einer Innenseite, insbesondere zumindest oder ausschließlich, einer, beispielsweise jeder, Schale können (die) zumindest (eine der) zwei Elektroden, insbesondere entlang und/oder quer einer Längsachse orientiert/erstrecken, integriert sein. In einer Innenseite jeder Schale können zumindest zwei, insbesondere Ablations-, Elektroden integriert sein, insbesondere entlang und/oder quer zu einer Längsachse orientiert/sich erstreckend.

Der Schalengreifer kann sich entlang einer Querachse erstrecken/ausdehnen und/oder eine maximale Ausdehnung aufweisen. Der Schalengreifer kann dazu eingerichtet sein, sich entlang einer Hochachse zu öffnen oder zu schließen. In einer Innenseite, insbesondere zumindest oder ausschließlich, einer, beispielweise jeder, Schale können, beispielsweise zusätzlich zu den zumindest zwei, insbesondere Ablations-, Elektroden, zumindest (eine der) drei, insbesondere Mess-, Elektroden, insbesondere entlang und/oder quer einer Längsachse orientiert/erstrecken und/oder in einem zweiten Elektrodenabstand zu den zumindest zwei Elektroden beabstandet, integriert sein. In einer Innenseite jeder Schale können zumindest drei, insbesondere Mess-, Elektroden, insbesondere entlang und/oder quer zu einer Längsachse orientiert/sich erstreckend, integriert sein.

Die zumindest zwei/drei Elektroden können in einem oberen/unteren Bereich entlang der Längsachse in der Innenseite der Schale angeordnet sein, während die zumindest drei/zwei Elektroden in einem unteren/oberen Bereich quer zu der Längsachse angeordnet sein können. Oder ausführlicher formuliert können die zumindest zwei Elektroden (z.B. Ablations-Elektroden) in einem oberen Bereich entlang der Längsachse in der Innenseite der Schale angeordnet sein, während die zumindest drei Elektroden (z.B. Mess-Elektroden) in einem unteren Bereich quer zu der Längsachse angeordnet sein können. Alternativ können die zumindest drei Elektroden (z.B. Mess-Elektroden) in einem unteren Bereich entlang der Längsachse in der Innenseite der Schale angeordnet sein, während die zumindest zwei Elektroden (z. B. Ablations-Elektroden) in einem oberen Bereich quer zu der Längsachse angeordnet sein können.

Die Kraftleitungseinheit kann als ein erster Zug- und Druckstab ausgebildet sein, der in dem Schaft angeordnet und mit der Gelenkanordnung verbunden ist. Eine Bewegung des ersten Zug- und Druckstabes kann zu einem Öffnen oder Schließen des Schalengreifers führen. Die Kraftleitungseinheit kann, insbesondere zusätzlich, als ein zweiter Zug- und Druckstab ausgebildet sein, der in dem Schaft angeordnet und beispielsweise ausschließlich mit dem Schalengreifer verbunden ist. Eine Bewegung des zweiten Zug- und Druckstabes kann zu einem, insbesondere in einer Längs-Querachsebene, Abwinkeln, beispielsweise einem beliebigen Einstellen eines Winkels, insbesondere zwischen inklusive 0° und 90°, des Schalengreifers gegenüber dem Schaft führen.

Die Kraftleitungseinheit und die Halteeinheit können als ein zusammenhängendes Formgedächtnismaterial, insbesondere eine Formgedächtnislegierung, ausgebildet sein. Die Halteeinheit kann als eine Fangstruktur, insbesondere ein Fangkörbchen, ausgebildet sein. Eine Bewegung der Kraftleitungseinheit kann zu einem Herausbewegen aus dem Schaft und Entfalten der Fangstruktur, insbesondere des Fangkörbchens, oder zu einem Zurückbewegen in den Schaft und Zusammenfalten der Fangstruktur, insbesondere des Fangkörbchens, führen.

Die Fangstruktur, insbesondere das Fangkörbchen, kann zwei einzelne und drei zusammenhängende Splines aufweisen. Die zumindest zwei, insbesondere Ablations-, Elektroden und/oder (zusätzlich den zumindest zwei Elektroden) zumindest drei, insbesondere Mess-, Elektroden können auf den Splines, insbesondere auf den zusammenhängenden Splines, integriert sein. Die drei zusammenhängenden Splines können in etwa die Form zweier, an deren Längsseiten zusammengefügter, stadionförmiger Splines aufweisen.

Gemäß einem zweiten Aspekt wird ein System zur laparoskopischen Ablation, insbesondere perivaskulären Neuromodulation, vorgeschlagen. Das System weist eine Vorrichtung gemäß dem ersten Aspekt auf. Das System weist eine mit der Vorrichtung verbundene oder verbindbare Signalgeneratoranordnung auf. Das System weist eine mit der Vorrichtung und/oder der Signalgeneratoranordnung verbundene oder verbindbare Steuer- und Auswerteeinheit auf.

Die Signalgeneratoranordnung kann einen ersten Signalgenerator zur Erzeugung eines ersten Signals, insbesondere eines Radiofrequenz (RF) -Signals, und einen zweiten Signalgenerator zur Erzeugung eines zweiten Signals, insbesondere eines Signals für eine gepulste Feldablation, aufweisen.

Dies hat den Vorteil, dass eine Kombination von RF-Signalen und gepulster Feldablation in einem System ermöglicht wird und die Gesamtprozedur der laparoskopischen Ablation verkürzt wird. Üblicherweise werden dafür zwei getrennte Systeme verwendet, die entweder RF-Signale oder gepulster Feldablationen zur Anwendung bringen und dafür abwechselnd in den Thorax eines Patienten eingebracht werden müssen.

Die Steuer- und Auswerteeinheit kann dazu eingerichtet sein, den ersten Signalgenerator und/oder den zweiten Signalgenerator zur Abgabe eines Signals anzusteuern.

Die Steuer- und Auswerteeinheit kann dazu ausgebildet sein, in Abhängigkeit von mindestens einer Elektroden-Temperatur den ersten Signalgenerator und/oder den zweiten Signalgenerator zur Abgabe eines Signals anzusteuern. Die Steuer- und Auswerteeinheit kann dazu ausgebildet sein, falls die Elektroden-Temperatur einen Temperatur-Grenzwert unterschreitet, den ersten Signalgenerator zur Abgabe eines Signals anzusteuern. Die Steuer- und Auswerteeinheit kann dazu ausgebildet sein, falls die Elektroden-Temperatur den Temperatur-Grenzwert annimmt oder überschreitet, den zweiten Signalgenerator zur Abgabe eines Signals anzusteuern.

Die Steuer- und Auswerteeinheit kann dazu eingerichtet sein, einen elektrischen Strom an die zumindest zwei Elektroden anzulegen und/oder über die zumindest drei Elektroden eine Spannung zu messen, wobei aus dem elektrischen Strom und der elektrischen Spannung eine Nervenaktivität des Gewebes und/oder die lokale Gewebeimpedanz bestimmt werden kann.

Gemäß einem dritten Aspekt wird ein Verfahren zur perivaskulären und/ oder perineuralen Neuromodulation vorgeschlagen. Das Verfahren umfasst ein Einführen eines Applikators in einen Patienten. Das Verfahren umfasst ein Positionieren eines distalen Endes des Applikators an einem Gefäß oder Gewebe oder Nerv des Patienten. Das Verfahren umfasst ein derartiges Positionieren eines distalen Endes des Applikators an einem Gefäß oder Gewebe oder Nerv des Patienten, dass mindestens zwei Elektroden des Applikators perivaskulär und/oder perineural mit einem Umfang des Gefäßes oder Gewebes oder Nervs in Kontakt stehen. Das Verfahren umfasst ein Durchführen einer Denervierung durch Energieabgabe über die mindestens zwei Elektroden. Die Energieabgabe erfolgt anhand eines Protokolls, das zumindest einen Puls einer gepulsten Feldablation (PFA) aufweist. Das Verfahren umfasst ein Herausnehmen des Applikators.

Durch das Verfahren gemäß dem dritten Aspekt kann eine irreversible Elektroporation ausgeführt werden.

Durch das Verfahren gemäß dem dritten Aspekt kann eine reversible Elektroporation ausgeführt werden.

Das distale Ende des Applikators kann alternativ an einem Gewebe, entsprechend zu der Positionierung an einem Gefäß oder Gewebe oder Nerv, positioniert werden. Zur Durchführung der Denervierung kann ein Generatorsystem vorgesehen sein. Das Generatorsystem kann elektrisch mit distal am Applikator / am distalen Ende des Applikators integrierten oder vorgesehenen zumindest zwei Elektroden verbunden sein.

Der Applikator kann durch ein laparoskopisches Verfahren eingeführt werden. Zusätzlich oder alternativ kann der Applikator durch ein laparoskopisches Verfahren herausgenommen werden.

Der laparoskopische Applikator kann durch einen Trokar in einen Torso des Patienten eingeführt werden. Der Applikator kann den Trokar aufweisen. Der Applikator kann ferner einen Schaft mit einem distalen und einem proximalen Ende umfassen. Das distale Ende kann beispielsweise mit einer Vielzahl von Elektroden ausgestattet sein.

Der Applikator kann durch ein offenes chirurgisches Verfahren eingeführt werden. Zusätzlich oder alternativ kann der Applikator durch ein offenes chirurgisches Verfahren herausgenommen werden.

Nach Einführen des Applikators kann eine geometrische Entfaltung eines distalen Endes des Applikators ausgelöst werden. Dadurch kann eine gefäß- oder gewebe- oder nervkonforme Form des distalen Endes erreicht werden. Die Entfaltung kann instruiert werden durch eine mechanische, magnetische oder materialbasierte Steuerung erfolgen.

Die Elektroden des Applikators können mit einem (Ziel-) Gefäß oder Gewebe oder Nerv in Kontakt gebracht werden.

Das Verfahren kann ferner ein Überprüfen einer Positionierung des Applikators durch eine lokale Impedanzmessung umfassen. Beispielsweise kann die Positionierung des Applikators bei Kontakt von zumindest zwei Elektroden mit dem Gefäß oder Gewebe oder Nerv als vollbracht angesehen werden. Im Anschluss kann das Überprüfen der Positionierung durchgeführt werden.

Das Protokoll kann beispielsweise ausschließlich PFA-Pulse aufweisen.

Das Protokoll kann eine Kombination aus PFA-Pulsen und Radiofrequenz (RF) -Pulsen aufweisen.

Das Verfahren kann ferner umfassen ein Auswählen, ob die Energieabgabe zur Denervierung anhand eines Protokolls mittels PFA-Pulsen oder anhand eines Protokolls mittels einer Kombination von PFA-Pulsen und RF-Pulsen durchgeführt werden soll.

Es kann eine einfache oder eine mehrfache Energieabgabe an gleichen oder verschiedenen Stellen des Gefäßes oder Gewebes oder Nervs erfolgen. Die Energieabgabe kann erfolgen mittels PFA-Pulsen oder mittels einer Kombination aus PFA-Pulsen und RF-Pulsen.

Durch das Durchführen der Denervierung kann eine perivaskuläre und/oder perineurale Neuromodulation ermöglicht werden oder auftreten. Dadurch ist eine Neuromodulation durch eine Gefäßwand hindurch nicht notwendig und kann vermieden werden.

Das Verfahren kann ferner, vor dem Durchführen der Denervierung, ein Quantifizieren einer Leitfähigkeit des Gefäßes oder Gewebes oder Nervs mittels mindestens zwei Stimulations- und Messelektroden umfassen. Die Quantifizierung der Leitfähigkeit kann erfolgen über eine Stimulations- und Messvorrichtung, welche distal und proximal zu mindestens einer Ablationselektrode angeordnete Stimulations- und Messelektroden aufweisen kann.

Das Verfahren kann ferner, nach dem Durchführen der Denervierung, ein Charakterisieren der Denervierung mittels mindestens zwei Stimulations- und Messelektroden umfassen. Die Charakterisierung der Denervierung kann erfolgen über eine Stimulations- und Messvorrichtung, welche distal und proximal zu mindestens einer Ablationselektrode angeordnete Stimulations- und Messelektroden aufweisen kann.

Während der Durchführung der beschriebenen Prozedur kann eine Temperatur an einer für die Ablation ausgewählten Ablationsstelle gemessen werden. Anders ausgedrückt, das Verfahren kann ferner ein Messen der Temperatur an der ausgewählten Ablationsstelle umfassen, während die Prozedur durchgeführt wird.

Weitere Merkmale, Eigenschaften, Vorteile und mögliche Abwandlungen werden für einen Fachmann anhand der nachstehenden Beschreibungen deutlich, in der auf die beigefügten Zeichnungen Bezug genommen ist.
Figur 1 ist eine schematische Darstellung eines biphasischen IRE-Pulses entsprechend einer Variante einer Ausführungsform.
Figur 2 ist eine schematische Darstellung eines Pulsprotokolls mit mehreren Bursts biphasischer Pulse entsprechend einer Variante einer Ausführungsform.
Figur 3 zeigt eine schematische Darstellung eines Prozedur-Protokolls, mit mindestens einem Burst biphasischer IRE-Pulse kombiniert mit mindestens einem RF-Energie Burst 120 entsprechend einer Variante einer Ausführungsform.
Figur 4 zeigt eine Ausführung des distalen Abschnitts eines laparoskopischen Applikators.
Figur 5 zeigt schematische Darstellungen zur Ansteuerung der zuvor beschriebenen Elektroden zur Durchführung eines Neuromodulationsverfahrens.
Figur 6 zeigt eine Ausführung des distalen Abschnitts eines laparoskopischen Applikators.
Figur 7 zeigt schematische Darstellungen von Elektroden-Anordnungen im Inneren einer Ansaugwanne und Ansteuerungsmöglichkeiten zur Durchführung einer Prozedur zur Neuromodulation.
Figur 8 zeigt eine Ausführung des distalen Abschnitts eines laparoskopischen Applikators.
Figur 9 zeigt eine weitere Ausführung des distalen Abschnitts eines laparoskopischen Applikators.
Figur 10 zeigt einen schematischen Ablauf zur Durchführung einer laparoskopischen Neuromodulation am Beispiel einer Denervations-Prozedur.

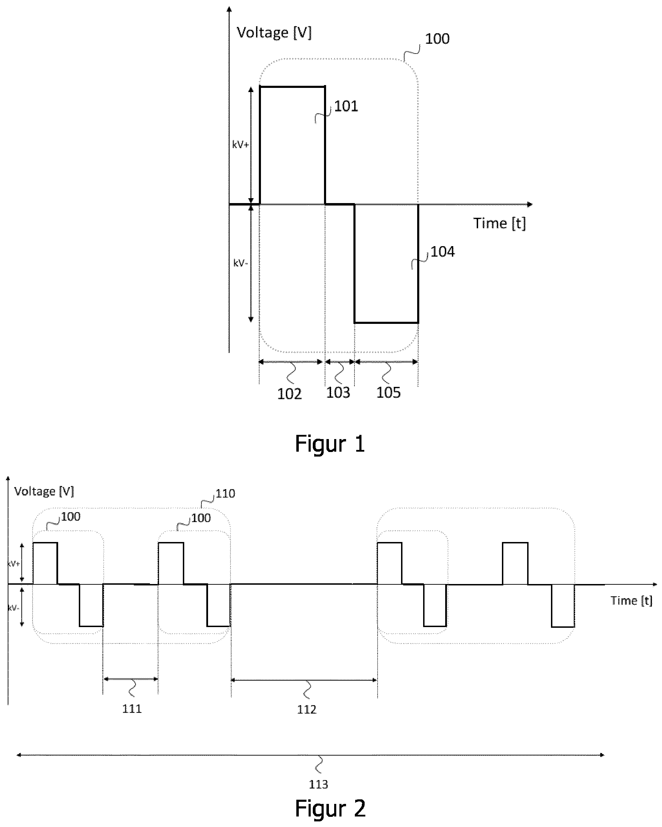

Figur 1 ist eine schematische Darstellung eines biphasischen IRE-Pulses (Irreversible Elektroporation) entsprechend einer Ausführungsvariante. Sie zeigt die Spannung V des biphasischen PFA-Pulses 100 als Funktion der Zeit t in einem IRE-Ablationsverfahren. Die vorliegende Ausführungsvariante bezieht sich auf einen zweiten Signalgenerator als IRE-Generator, der als Spannungsquelle konfiguriert ist. Folglich werden die IRE-Signale hier in Form ihrer Spannungen beschrieben. Der biphasische IRE-Puls 100 umfasst einen positiven Puls 101 und einen negativen Puls 104, wobei sich die Begriffe "positiv" und "negativ" auf eine unabhängig gewählte Polarität zweier für die Ablation angesteuerten Elektroden beziehen, zwischen denen der biphasische Puls angelegt wird. Die Amplitude des positiven Pulses 101 wird mit kV+ bezeichnet und dauert über die Zeit 102 an. Analog wird die Amplitude des negativen Pulses 104 mit kV- bezeichnet und weist eine Dauer 105 auf. Zwischen den beiden Puls-Phasen 101 und 104 liegt eine Verzögerungszeit 103. Sowohl die beiden zeitlichen Puls-Breiten 102 und 105 als auch die Amplitude kV+ und kV- sind unabhängig voneinander konfigurierbar und können daher in einer exemplarischen Ausführung der Erfindung variieren.

Figur 2 ist eine schematische Darstellung eines Pulsprotokolls mit mehreren Bursts biphasischer Pulse entsprechend einer Ausführungsvariante. Über die Dauer der kompletten IRE-Prozedur 113 werden die Pulse 100 in Form eines oder mehrerer **Bursts bzw.** Pulspakete 110 abgegeben. Jeder Burst 110 umfasst dabei eine definierte Anzahl N an biphasischen Pulsen 100, wobei die Pulse durch ein zeitliches Intervall 111 separiert sind. Zwischen der Abgabe der einzelnen Bursts 110 liegt wiederum eine Verzögerungszeit 112.

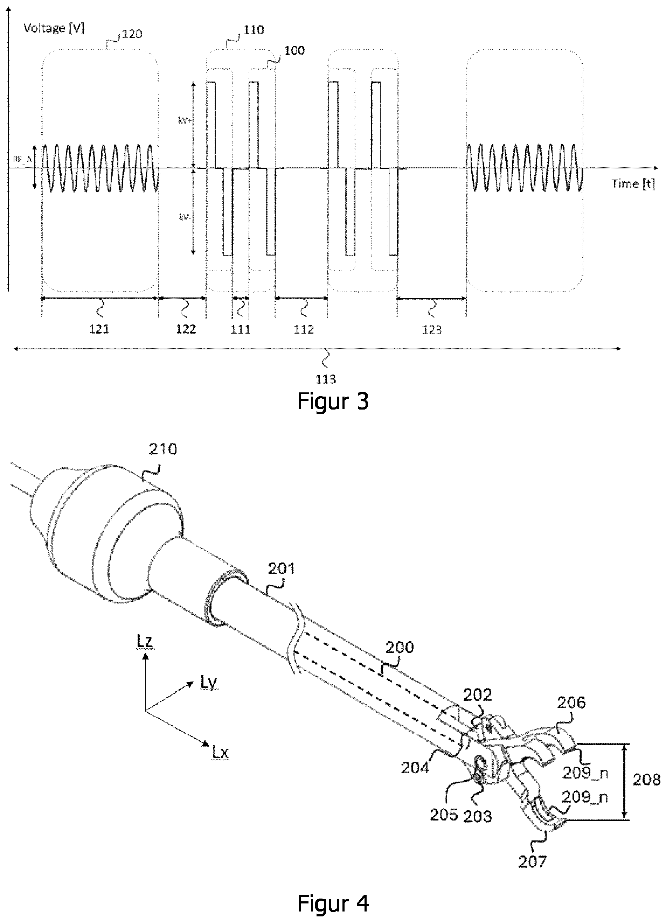

Figur 3 zeigt eine schematische Darstellung eines Prozedur-Protokolls mit mindestens einem Burst biphasischer IRE-Pulse kombiniert mit mindestens einem RF-Energie Burst 120 entsprechend einer Ausführungsvariante. Über die Dauer der kompletten kombinierten Prozedur 113 werden die RF-Energie sowie die IRE-Pulse in Form einer oder mehrerer Bursts 120 und 110 abgegeben. Jeder IRE-Burst umfasst dabei eine definierte Anzahl N an bipolaren Pulsen 100, wobei die Pulse durch ein zeitliches Intervall 111 separiert sind. Der RF-Burst ist beschrieben durch ein sinusförmiges Signal mit Amplitude RF_A und einer Dauer 121. Im Anschluss an einen RF-Burst folgt eine Verzögerungszeit 122. Sowohl die Dauer des RF-Bursts als auch die anschließende Verzögerungszeit 122 können basierend auf der aktuell gemessenen Temperatur an den Ablations-Elektroden geregelt werden. Zwischen der Abgabe der einzelnen IRE-Bursts 110 liegt eine Verzögerungszeit 112. Zwischen einem IRE-Burst und einer erneuten Abgabe des RF-Burst liegt eine Verzögerungszeit 123.

Figur 4 zeigt eine Ausführungsvariante des distalen Abschnitts eines laparoskopischen Applikators. Der Applikator weist einen Schaft 201 auf, der mit Hilfe eines Trokars 210 durch den Torso eines Patienten eingeführt werden kann. Im Inneren des Schafts 201 verläuft ein Zug- und Druckstab 200, der entlang einer Längsachse Lx frei und relativ zu dem Schaft beweglich ist. Am distalen Ende des Applikators befinden sich ein Drei-Fingergreifer 206, 207 aufweisend ein Zwei-Fingerteil 206 und Ein-Fingerteil 207. Jeder der drei Finger weist eine Innenseite, insbesondere eine konkave Ausbuchtung auf. Der Drei-Fingergreifer 206, 207 ist dazu eingerichtet, eine zylindrische Gewebestruktur zu greifen, deren Umfang um eine Querachse Ly ausgebildet ist. Die zylindrische Gewebestruktur weist eine Längsausdehnung auf, die parallel zur der Querachse Ly des laparoskopischen Applikators orientiert ist. Zur Realisierung des Greifmechanismus ist der Drei-Fingergreifer 206, 207 über eine Gelenkanordnung 202, 203, 204, 205 mit dem äußeren Schaft 201 sowie mit dem inneren Zug- und Druckstabs 200 derart gelagert, dass eine Bewegung des Zug- und Druckstabs 200 entlang der Längsachse Lx zu einem Greifen des Drei-Fingergreifers führt. Ein Greifabstand 208 ist damit variabel einstellbar und somit an verschiedene Gewebestrukturen adaptierbar. Auf den konkaven Ausbuchtungen des Drei-Fingergreifers 206, 207 sind eine Vielzahl von Elektroden 209_n integriert. Jede konkave Ausbuchtung weist eine Elektrode auf. Jeder der Elektroden 209_n ist über elektrische Kontaktierungen, die im Inneren des Schafts 201 verläuft, mit einer Auswerte- und Steuereinheit (nicht dargestellt) sowie dem Generatorsystem verbunden (nicht dargestellt). Diese Elektroden sind jeweils einer äußeren Umgebung ausgesetzt, wohingegen die elektrischen Verbindungen zur Steuereinheit und dem Generatorsystem zur äußeren Umgebung hin isoliert sind. In der vorliegenden Ausführungsvariante sind die Elektroden als Ablationselektroden ausgebildet, womit eine laparoskopische Ablation ermöglicht wird.

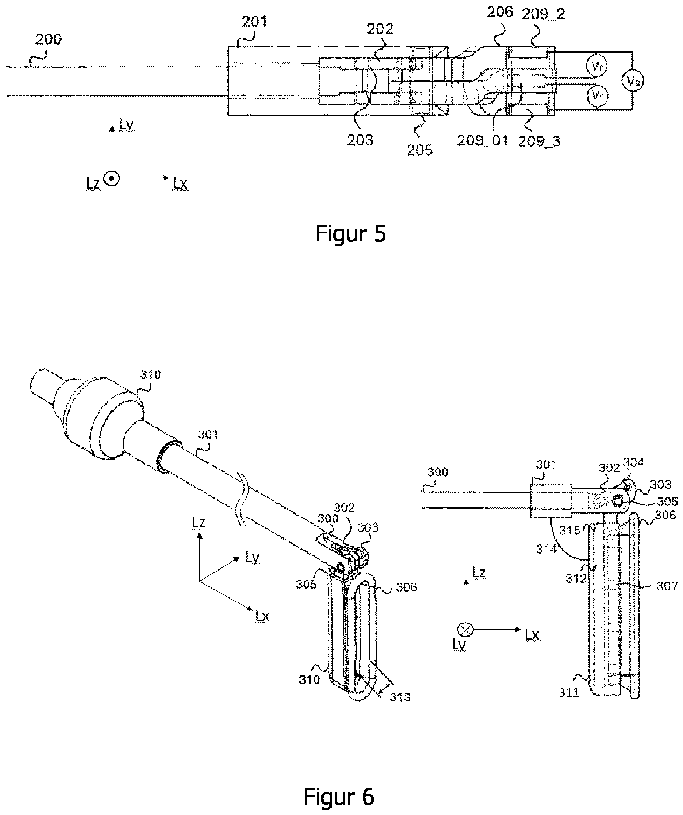

Figur 5 zeigt schematische Darstellungen zur Ansteuerung der zuvor beschriebenen Elektroden 209_n zur Durchführung eines Neuromodulationsverfahrens. In dieser Ausführung befinden sich die Elektroden 209_2 und 209_3 auf dem Zwei-Fingerteil 206 und Elektroden 209_1 auf dem Ein-Fingerteil 207. Ansteuerungsmöglichkeit 1 sieht die Erzeugung eines elektrischen Feldes in radialer Richtung vor. Dazu werden die beiden Spannungen Vr zwischen dem Elektrodenpaar 209_2 und 209_1 sowie zwischen dem Elektrodenpaar 209_3 und 209_1 angelegt. Bei Kontakt aller Elektroden mit einer Gewebestruktur ergibt sich ein radial orientierter Stromfluss. Bei Ansteuermöglichkeit 2 wird ein in Querachse Ly orientierter Stromfluss induziert, indem ein elektrisches Feld mittels einer Spannung Va zwischen dem Elektrodenpaar 209_2 und 209_3 angelegt wird. Elektroden 209_1 ist hierbei ohne Funktion und nimmt einen elektrisch floatenden Zustand ein.

Figur 6 zeigt eine weitere Ausführungsvariante des distalen Abschnitts eines laparoskopischen Applikators. Der Applikator weist einen äußeren Schaft 301 auf, der mit Hilfe eines Trokars 310 durch den Torso eines Patienten eingeführt werden kann. Im Inneren des Schafts 301 verläuft ein Zug- und Druckstab 300, der entlang einer Längsachse Lx frei und relativ zu dem Schaft 301 beweglich ist. Am distalen Ende des Applikators befindet sich eine Ansaugeinheit 311 mit einer flexiblen atraumatischen Ansaugwanne 306, die mittels Unterdruck Kontakt zu einer zylindrischen Gewebestruktur herstellen kann. Die Ansaugwanne 311 ist über eine Gelenkanordnung 302, 303, 304, 305 mit dem Schaft 301 und dem Zug- und Druckstab 300 derart verbunden, dass ein Winkel 314 zwischen der Längsachse Lx des Schafts 301 und der Ansaugeinheit 311 frei eingestellt werden kann. Mit Hilfe des einstellbaren Winkels kann sowohl eine Einführung durch den Trokar 310 realisiert als auch ein optimaler Kontakt zur Gewebestruktur ermöglicht werden. Im Inneren der Ansaugwanne 306 befindet sich eine Vielzahl von Ansauglöchern 307, die mit einem Ansaugkanal 312 verbunden sind. Die Ansaugwanne weist eine Wannenbreite 313 auf. Am Ausgang des Ansaugkanals 312 befindet sich ein Vakuumanschluss 315, der eine Verbindung zu einer externen Vakuumpumpe herstellt, um im Inneren der Ansaugwanne 306 einen Unterdruck zu erzeugen.

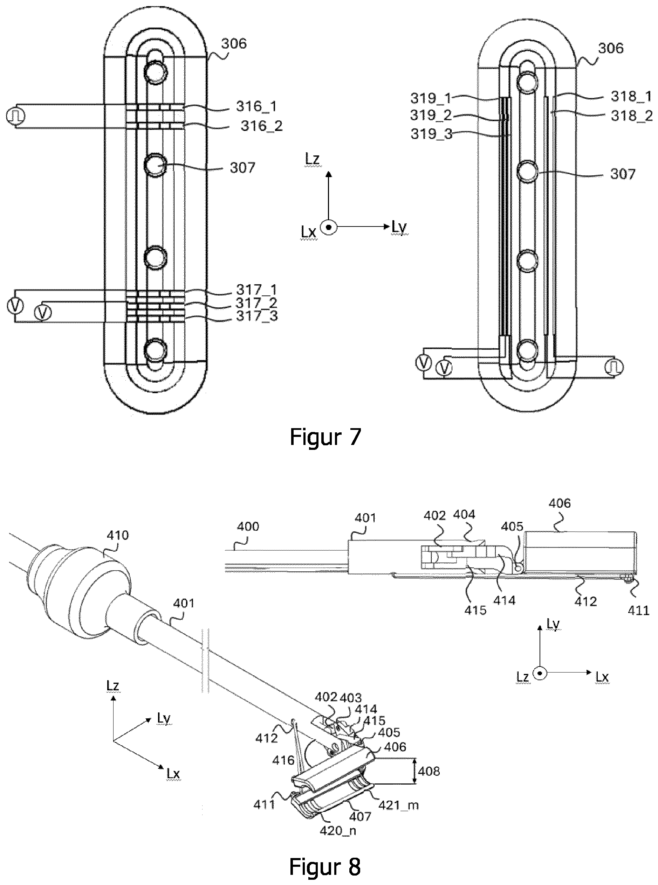

Figur 7 zeigt schematische Darstellungen von Elektroden-Anordnungen im Inneren der Ansaugwanne 306 und Ansteuerungsmöglichkeiten zur Durchführung einer Prozedur zur Neuromodulation. Bei Elektrodenanordnung 1 (links) gibt es zwei Ablations- und Stimulationselektroden 316_1 und 316_2, sowie drei Messelektroden 317_1, 317_2 und 317_3. Alle Elektroden sind derart angeordnet, dass sie senkrecht zur einer Längsachse einer angesaugten zylindrischen Gewebestruktur orientiert sind. Über die beiden Elektroden 316_1 und 316_2 kann zu Beginn der Prozedur eine Baseline-Stimulation der Nerven durchgeführt werden, indem ein definierter elektrischer Strom an dieses Elektrodenpaar angelegt wird. Mit Hilfe der drei Messelektroden 317_1, 317_2 und 317_3 wird bei gleichzeitiger Stimulation die Reizweiterleitung der Nerven gemessen. Dazu werden zwei bipolare Spannungen über das Elektrodenpaar 317_1 und 317_2 sowie über das Elektrodenpaar 317_2 und 317_3 erfasst und verarbeitet. Im Anschluss an diese Baseline-Messung erfolgt eine bipolare Ablation über das Elektrodenpaar 316_1 und 316_2. Zur Charakterisierung der Neuromodulation wird im Anschluss der Ablation erneut die Nervenleitfähigkeit, wie zuvor beschreiben, gemessen und analysiert. Der Ablauf dieser Prozedur ist in Elektrodenanordnung 2 (rechts) identisch. Sie unterscheidet sich jedoch in der Anordnung der Elektroden innerhalb der Ansaugwanne 306. In dieser Konfiguration sind alle Elektroden parallel zur einer Längsachse einer angesaugten zylindrischen Gewebestruktur orientiert. Rechts der Vielzahl von Ansauglöchern 307 liegen die beiden Ablations- und Stimulationselektroden 318_1 und 318_2, links der Vielzahl von Ansauglöchern 307 befinden sich die Messelektroden 319_1, 319_2 und 319_3.

Figur 8 zeigt eine Ausführungsvariante des distalen Abschnitts eines laparoskopischen Applikators. Er weist einen äußeren Schaft 401 auf, der mit Hilfe eines Trokars 410 durch den Torso eines Patienten eingeführt werden kann. Im Inneren des Schafts 401 verläuft ein erster Zug- und Druckstab 400, der entlang einer Längsachse Lx frei beweglich ist. Am distalen Ende des Applikators befinden sich ein Zwei-Halbschalengreifer mit einer ersten Halbschale 406 und einer zweiten Halbschale 407, die jeweils eine konkave Ausbuchtung aufweisen und dazu eingerichtet sind, eine zylindrische Gebestruktur greifen zu können. Zur Realisierung des Greifmechanismus ist der Zwei-Halbschalengreifer über eine Gelenkanordnung 402, 403, 404, 405, 414, 415 mit dem äußeren Schaft 401 sowie mit dem inneren Zug- und Druckstab 400 derart gelagert, dass eine Bewegung des Zug- und Druckstabs 400 entlang der Längsachse Lx zu einem Klemmen der ersten 406 und zweiten Halbschalen 407 führt. Ein realisierter Klemmabstand 408 ist hierdurch variabel einstellbar und an verschiedene Gewebestrukturen adaptierbar. Im Inneren des Schafts 401 verläuft ein zweiter Zug- und Druckstab 412, der mit der ersten 406 und zweiten Halbschale 407 derart verbunden ist, dass ein Winkel 416 zwischen der Längsachse Lx des Schafts 401 und dem Zwei-Halbschalengreifer eingestellt werden kann. Mit Hilfe des einstellbaren Winkels 416 kann sowohl eine Einführung durch den Trokar 410 realisiert werden als auch ein optimaler Kontakt zur Gewebestruktur ermöglicht werden. Auf den konkaven Ausbuchtungen der ersten 406 und zweiten Halbschale 407 befinden sich eine Vielzahl von Ablations- und Stimulationselektroden 420_n sowie Messelektroden 421_m, die individuell über elektrische Kontaktierungen, die im Inneren des Schafts 401 verlaufen, mit der Auswerte- und Steuereinheit sowie dem Generatorsystem verbunden sind (beide nicht dargestellt). Diese Elektroden sind jeweils der äußeren Umgebung ausgesetzt, während die elektrischen Verbindungen zur Steuereinheit und dem Generatorsystem von der äußeren Umgebung isoliert sind. Am Beispiel von zwei Ablations- und Stimulationselektroden und drei Messelektroden erfolgt die Ansteuerung analog zur Beschreibung in Figur 7.

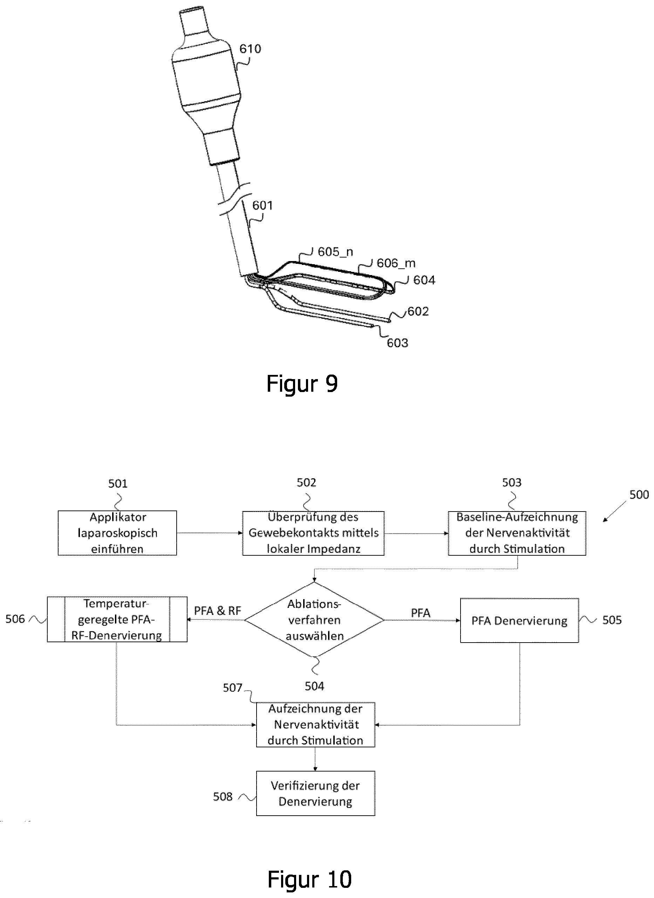

Figur 9 zeigt eine weitere Ausführungsvariante des distalen Abschnitts eines laparoskopischen Applikators. Der Applikator weist einen äußeren Schaft 601 auf, der mit Hilfe eines Trokars 610 durch den Torso eines Patienten eingeführt werden kann. Im Inneren des Schafts 601 verläuft eine Fangstruktur, bspw. ein Fangkörbchen, beispielsweise aus einem Formgedächtnismaterial, das aus drei zusammenhängenden Splines 604 und zwei einzelnen Splines 602 und 603 zusammengesetzt ist. Die gesamte Fangstruktur ist dabei derart elastisch, dass sie vollständig in das Innere des Schafts 601 hineingezogen werden kann. Sie ist dazu eingerichtet, im dargestellten Zustand eine zylindrische Gewebestruktur zu greifen und umschlingen zu können.

Auf den drei zusammenhängenden Splines 604 befinden sich jeweils eine Vielzahl von Stimulations- und Ablationselektroden 605_n sowie eine Vielzahl von Messelektroden 606_m. Jede der Vielzahl von Elektroden 605_n, 606_m sind individuell über elektrische Kontaktierungen, die im Inneren des Schafts 601 verlaufen, mit der Auswerte- und Steuereinheit sowie dem Generatorsystem verbunden (beide nicht dargestellt). Die Elektroden sind jeweils der äußeren Umgebung ausgesetzt, während die elektrischen Verbindungen zur Auswerte- und Steuereinheit und dem Generatorsystem von der äußeren Umgebung isoliert sind. Am Beispiel von zwei Ablations- und Stimulationselektroden und drei Messelektroden erfolgt die Ansteuerung analog zur Beschreibung in Figur 7.

Figur 10 zeigt einen schematischen Ablauf 500 zur Durchführung einer laparoskopischen Neuromodulation am Beispiel einer Denervations-Prozedur. Einer oder mehrere der in Bezug auf Figur 10 beschriebenen Schritte kann unter Verwendung eines laparoskopischen Applikators durchgeführt werden, wie er beispielhaft in Bezug auf die Figuren 4, 6, 8 und 9 beschrieben wurde. Im ersten Schritt 501 wird ein Applikator-System, insbesondere ein laparoskopischer Applikator, laparoskopisch in den Patienten eingeführt. Wie in Bezug auf die Figuren 4, 6, 8 und 10 beispielhaft beschrieben, kann der laparoskopische Applikator einen Trokar aufweisen, durch welchen ein Schaft des laparoskopischen Applikators in den Torso eines Patienten eingeführt werden kann. Nach Positionierung des Applikators wird durch Messung der lokalen Impedanz an den Ablationselektroden überprüft, ob diese einen ausreichend guten Kontakt zum Gewebe haben (Schritt 502). Im nächsten Schritt 503 erfolgt die Aufzeichnung des Ausgangswertes der Nervenleitfähigkeit, beispielsweise durch die zuvor in Bezug auf Figur 7 beschriebenen Mechanismen. Beispielsweise kann durch das Anlegen eines definierten elektrischen Stroms an einer zu einer Ablationselektrode distalen oder proximalen Elektrode ein Stimulationspuls an einen oder mehrere Nerven abgegeben. Eine resultierende bipolare Spannung wird bei gleichzeitiger Stimulation an Messelektrodenpaaren gemessen und zur Charakterisierung der Nervenleitfähigkeit genutzt. Anschließend kann in Schritt 504 zwischen zwei Arten von Prozeduren oder Eingabeoptionen gewählt werden: eine Denervation mittels gepulster Feldablation (PFA) oder eine Kombinations-Prozedur aus PFA und RF-Energie. Im Falle der PFA-Denervation (Schritt 505) erfolgt die Prozedur, insbesondere eine Energieabgabe, anhand eines wie in Figur 2 beschriebenen beispielhaften Protokolls mittels biphasischer IRE-Pulse. Wird eine PFA-RF-Kombinationsprozedur (506) gewählt, erfolgt die Prozedur, insbesondere eine Energieabgabe, anhand eines wie in Figur 3 beispielhaft beschriebenen Kombinations-Protokolls. Unabhängig von der Wahl des Denervations-Verfahrens folgt daraufhin eine erneute Aufzeichnung der Nervenaktivität (507) zur Charakterisierung der Denervation (508) wie in Figur 7, Figur 8 und Figur 9 beschrieben.

Neben einem vorteilhaften Applikator werden hierin eine Laparoskopie-basierte Neuromodulation von Nerven durch eine kombinierte Prozedur aus aufeinanderfolgenden IRE und Radiofrequenz (RF)-Sequenzen beschrieben. Auf diese Weise kann unter kontinuierlicher Überwachung der Temperatur gezielt die Gewebeleitfähigkeit erhöht werden. Damit kann der Erfolg der irreversiblen Elektroporation maximiert und gleichzeitig das Auftreten von potenziell schädigenden hohen Stromdichten reduziert werden. Durch Nutzung des Applikators muss nicht durch die Gefäßwand hindurch abladiert werden.

## Patentansprüche

1. Vorrichtung zur Anwendung in der laparoskopischen Chirurgie, aufweisend
- einen Schaft, der ein distales und ein proximales Ende aufweist,
- eine Halteeinheit, die an dem distalen Ende des Schafts angeordnet und dazu eingerichtet ist, ein Gewebe zu halten und/oder loszulassen,
- zumindest zwei in der Halteeinheit integrierte Elektroden,
- eine Kraftleitungseinheit, die an dem Schaft angeordnet, mit der Halteeinheit verbunden und dazu eingerichtet ist, relativ zu dem Schaft bewegt zu werden,
- eine elektrische Leitung, die in dem Schaft angeordnet, mit den zumindest zwei Elektroden verbunden und dazu eingerichtet ist, ein über das proximale Ende des Schafts zu empfangendes elektrisches Signal an die zumindest zwei Elektroden weiterzuleiten.

2. Vorrichtung nach Anspruch 1, aufweisend eine Gelenkanordnung, die zwischen der Halteeinheit und dem distalen Ende des Schafts angeordnet und dazu eingerichtet ist, das distale Ende des Schaftes mit der Halteeinheit, insbesondere beweglich, zu verbinden, wobei eine Bewegung der Kraftleitungseinheit zu einer Bewegung der Halteeinheit und/oder zu dem Halten und/oder Loslassen des Gewebes mittels der Kraftleitungseinheit führt; und/oder
wobei die Halteeinheit als ein Fingergreifer, insbesondere ein Drei-Fingergreifer, und die Kraftleitungseinheit als Zug- und Druckstab ausgebildet ist, wobei eine Bewegung des Zug- und Druckstabs zu einem Öffnen oder Schließen des Fingergreifers führt, um das Gewebe zu greifen oder loszulassen.

3. Vorrichtung nach Anspruch 2, wobei Finger des Fingergreifers jeweils eine, insbesondere in einem Schließ-Zustand des Fingergreifers, zu dem Gewebe orientierte Innenfläche aufweisen, auf die zumindest eine der zumindest zwei Elektroden, insbesondere eine von einer Vielzahl von Elektroden, integriert ist.

4. Vorrichtung nach Anspruch 1 oder 2, wobei die Halteeinheit als Ansaugeinheit ausgebildet ist, wobei eine Bewegung der Kraftleitungseinheit zu einem Abwinkeln der Ansaugeinheit gegenüber dem Schaft führt, und/oder die Ansaugeinheit dazu eingerichtet ist, ein Gewebe anzusaugen oder sich vom Gewebe zu lösen.

5. Vorrichtung nach Anspruch 4, wobei die Ansaugeinheit aufweist:
- eine Ansaugwanne,
- zumindest ein Ansaugloch, das in der Ansaugwanne angeordnet ist,
- einen Ansaugkanal, der mit dem zumindest einen Ansaugloch verbunden ist, und/oder
- einen an einem Ende des Ansaugkanals vorgesehenen Ansauganschluss, über den eine externe Vakuumpumpe anschließbar ist, um einen Unterdruck in der Ansaugwanne zu erzeugen und das Gewebe an die Ansaugwanne anzusaugen.

6. Vorrichtung nach Anspruch 5, wobei die Ansaugwanne entlang einer Hochachse oval, insbesondere stadionförmig oder kreisrund, ausgebildet ist und die zumindest zwei Elektroden entlang der Hochachse und/oder quer zu der Hochachse in der Ansaugwanne angeordnet sind; und/oder
wobei die Ansaugwanne entlang einer Hochachse oval, insbesondere stadionförmig oder kreisrund, ausgebildet ist und zumindest drei Elektroden entlang der Hochachse und/oder quer zu der Hochachse in der Ansaugwanne, insbesondere von den zumindest zwei Elektroden beabstandet, angeordnet sind.

7. Vorrichtung nach Anspruch 1 oder 2, wobei die Halteeinheit als ein Schalengreifer, insbesondere ein Zwei-Halbschalengreifer, ausgebildet ist, wobei eine Bewegung der Kraftleitungseinheit zu einem Abwinkeln des Schalengreifers gegenüber dem Schaft und/oder zum Öffnen und/oder zum Schließen des Schalengreifers führt.

8. Vorrichtung nach Anspruch 7, wobei der Schalengreifer sich entlang einer Querachse erstreckt und dazu eingerichtet ist, sich entlang einer Hochachse zu öffnen oder zu schließen, wobei in einer Innenseite zumindest einer Schale die zumindest zwei Elektroden, insbesondere entlang einer Längsachse, integriert sind; und/oderwobei der Schalengreifer sich entlang einer Querachse erstreckt und dazu eingerichtet ist, sich entlang einer Hochachse zu öffnen oder zu schließen, wobei in einer Innenseite zumindest einer Schale zumindest drei Elektroden, insbesondere entlang und/oder quer zu einer Längsachse, von den zumindest zwei Elektroden beabstandet, integriert sind.

9. Vorrichtung nach Anspruch 7 oder 8, wobei die Kraftleitungseinheit als ein erster Zug- und Druckstab ausgebildet ist, der in dem Schaft angeordnet und mit der Gelenkanordnung verbunden ist, wobei eine Bewegung des ersten Zug- und Druckstabes zu einem Öffnen oder Schließen des Schalengreifers führt, und/oder die Kraftleitungseinheit als ein zweiter Zug- und Druckstab ausgebildet ist, der in dem Schaft angeordnet und, insbesondere ausschließlich, mit dem Schalengreifer verbunden ist, wobei eine Bewegung des zweites Zug- und Druckstabes zu einem Abwinkeln des Schalengreifers gegenüber dem Schaft führt.

10. Vorrichtung nach einem der Ansprüche 1 bis 9, wobei die Kraftleitungseinheit und die Halteeinheit als zusammenhängendes Formgedächtnismaterial, insbesondere Formgedächtnislegierung, ausgebildet sind, und die Halteeinheit eine Fangstruktur, insbesondere Fangkörbchen bildet, wobei eine Bewegung der Kraftleitungseinheit zu einem Zurückbewegen in den Schaft und einem Zusammenfalten der Fangstruktur, insbesondere des Fangkörbchens, oder zu einem Herausbewegen aus dem Schaft und einem Entfalten der Fangstruktur, insbesondere des Fangkörbchens, führt.

11. Vorrichtung nach Anspruch 10, wobei die Fangstruktur zwei einzelne und drei zusammenhängende Splines aufweist, wobei die zumindest zwei Elektroden und/oder zumindest drei Elektroden auf den Splines, insbesondere auf den zusammenhängenden Splines, integriert sind.

12. System zur laparoskopischen Ablation, aufweisend:
- eine Vorrichtung nach einem der Ansprüche 1 bis 11;
- eine mit der Vorrichtung verbundene oder verbindbare Signalgeneratoranordnung; und
- eine mit der Vorrichtung und/oder der Signalgeneratoranordnung verbundene oder verbindbare Steuer- und Auswerteeinheit.

13. System nach Anspruch 12, wobei die Signalgeneratoranordnung einen ersten Signalgenerator zur Erzeugung eines Radiofrequenz, RF, -Signals und einen zweiten Signalgenerator zur Erzeugung eines Signals für eine gepulste Feldablation aufweist; und/oderwobei die Steuer- und Auswerteeinheit ausgebildet ist, den ersten Signalgenerator und/oder den zweiten Signalgenerator zur Abgabe eines Signals anzusteuern.

14. System nach Anspruch 13, wobei die Steuer- und Auswerteeinheit ausgebildet ist, in Abhängigkeit von mindestens einer Elektroden-Temperatur den ersten Signalgenerator und/oder den zweiten Signalgenerator zur Abgabe eines Signals anzusteuern,
wobei die Steuer- und Auswerteeinheit insbesondere ausgebildet ist, falls die Elektroden-Temperatur einen Temperatur-Grenzwert unterschreitet, den ersten Signalgenerator zur Abgabe eines Signals anzusteuern und, falls die Elektroden-Temperatur den Temperatur-Grenzwert annimmt oder überschreitet, den zweiten Signalgenerator zur Abgabe eines Signals anzusteuern.

15. System nach einem der Ansprüche 12 bis 14, wobei die Steuer- und Auswerteeinheit dazu eingerichtet ist, einen elektrischen Strom an die zumindest zwei Elektroden anzulegen und/oder über die zumindest drei Elektroden eine Spannung zu messen, wobei aus dem elektrischen Strom und der elektrischen Spannung eine Nervenaktivität des Gewebes und/oder die lokale Gewebeimpedanz bestimmbar ist oder bestimmt wird.
